(19) Europäisches Patentamt
European Patent Office
Office européen des brevets

(11) Veröffentlichungsnummer: **0 070 485**
**B1**

(12) **EUROPÄISCHE PATENTSCHRIFT**

(45) Veröffentlichungstag der Patentschrift: **18.03.87**

(51) Int. Cl.⁴: **C 07 C 141/02**

(21) Anmeldenummer: **82106201.5**

(22) Anmeldetag: **10.07.82**

(54) **Verfahren zur Herstellung von omega-Fluorsulfato-perfluoralkansäurederivaten.**

(30) Priorität: **16.07.81 DE 3128119**

(43) Veröffentlichungstag der Anmeldung: **26.01.83 Patentblatt 83/4**

(45) Bekanntmachung des Hinweises auf die Patenterteilung: **18.03.87 Patentblatt 87/12**

(84) Benannte Vertragsstaaten: **BE CH DE FR GB IT LI NL**

(56) Entgegenhaltungen:
**US-A-4 181 679**

**INORGANIC CHEMISTRY, Band 3, Nr. 2, Februar 1964, Seiten 287-288, Washington, USA**
**J.Fluorine Chemistry 16, S.65(1980)**

(73) Patentinhaber: **HOECHST AKTIENGESELLSCHAFT, Postfach 80 03 20, D-6230 Frankfurt am Main 80 (DE)**

(72) Erfinder: **Siegemund, Günter, Dr., Frankfurter Strasse 21, D-6238 Hofheim am Taunus (DE)**
Erfinder: **Schwertfeger, Werner, Dr., Erlenstrasse 8, D-6306 Langgöns (DE)**

**EP 0 070 485 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

ω-Fluorsulfato-perfluoralkansäurederivate sind Verbindungen der allgemeinen Formel:

$$FSO_2-O-R_f-CX,$$

worin $R_f$ = unverzweigter oder verzweigter Perfluoralkylenrest, und-

X =

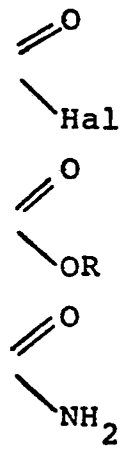

(Hal = Halogen)

(R = organischer Rest)

$\equiv N$, etc.

ω-Fluorsulfato-perfluorcarbonsäurederivate der Formel I

$$FSO_2-O-(CF_2)_m-(CF_2-O-\underset{CF_3}{\underset{|}{CF}})_n-COA \qquad (I)$$

worin A = Halogen, vorzugsweise Cl oder F, insbesondere F,
oder die Gruppe OR (R = organ. Rest, insbesondere
$CH_3$ oder $C_2H_5$),
m = 1 - 10, und
n = 0 - 10

können nach dem Verfahren der Patentanmeldung P 30 34 538.1 HOE 80/F 196) dadurch erhalten werden, daß man a) ω-H-perfluorcarbonsäurehalogenide der Formel II

$$H(CF_2)_m-(CF_2-O-\underset{CF_3}{\underset{|}{CF}})_n-COA' \qquad (II)$$

worin A' = Halogen und
m und n die gleiche Bedeutung wie in Formel I besitzen,
in einem Elektrolyten aus Fluorsulfonsäure und einem Alkalifluorsulfonat
unter Verwendung von Platin oder Metallen der Platingruppe und/oder glasartigem Kohlenstoff als Anodenmaterialien und von üblichen, jedoch unter den Elektrolysebedingungen stabilen Kathodenmaterialien elektrolysiert,
die dabei gebildeten ω-Fluorsulfato-perfluorcarbonsäurehalogenide der Formel III

$$FSO_2-O-(CF_2)_m-(CF_2-O-\underset{CF_3}{\underset{|}{CF}})_n-COA' \qquad (III)$$

worin A' die gleiche Bedeutung wie in Formel II und m und n die gleiche Bedeutung wie in den Formeln I und II besitzen,
isoliert und - zur Herstellung der entsprechenden Ester- b) mit einer organischen Hydroxylverbindung der Formel IV
ROH (IV)
worin R die bei Formel I genannte Bedeutung besitzt,
zu ω-Fluorsulfato-perfluorcarbonsäurederivaten der Formel I mit A = OR verestert.

Die Ausgangsverbindungen für dieses Verfahren - also die ω-H-perfluorcarbonsäurehalogenide der Formel II - können z.B. nach folgenden bekannten Verfahrensweisen erhalten werden: 1. J.Am. Chem. Soc. 74 (1952), 1426:

Aus Ammoniak und Tetrafluorethylen wird in Gegenwart von Kupferacetat zunächst das folgende Triazinderivat hergestellt:

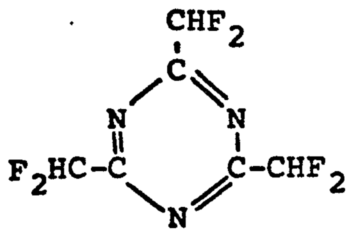

welches dann durch Erhitzen mit wässriger Natronlauge in das Natriumsalz der Difluoressigsäure $HCF_2$-COONa überführt wird. Daraus sind die Säurehalogenide der Formel II (mit m = 1 und n = 0) nach bekannten Methoden erhältlich.

US-PS 2 559 629:

Herstellung von aliphatischen Polyfluorcarbonsäuren und ihren Salzen durch Oxidation von Polyfluoralkanolen mit Permanganat:

$$H(CX_2CX_2)_nCH_2OH \xrightarrow{\text{Oxidation}} H(CX_2CX_2)_nCOOH$$

X = Cl, F, mit mindestens der Hälfte der Reste X = F n = 1 - 3

Die Ausgangsverbindungen für diese Oxidation werden aus Ethylenderivaten $CX_2 = CX_2$ und Methanol hergestellt.

Aus den erhaltenen freien Säuren werden die Säurehalogenide auf bekannte Weise gewonnen; die Verbindungen mit allen Resten X = F stellen die Verbindungen der Formel II mit m = gerade Zahlen und n = 0 dar.

J. Org. Chem. Vol. 42, Nr. 25 (1977), 4055: beschreibt u.a. folgende Reaktion:

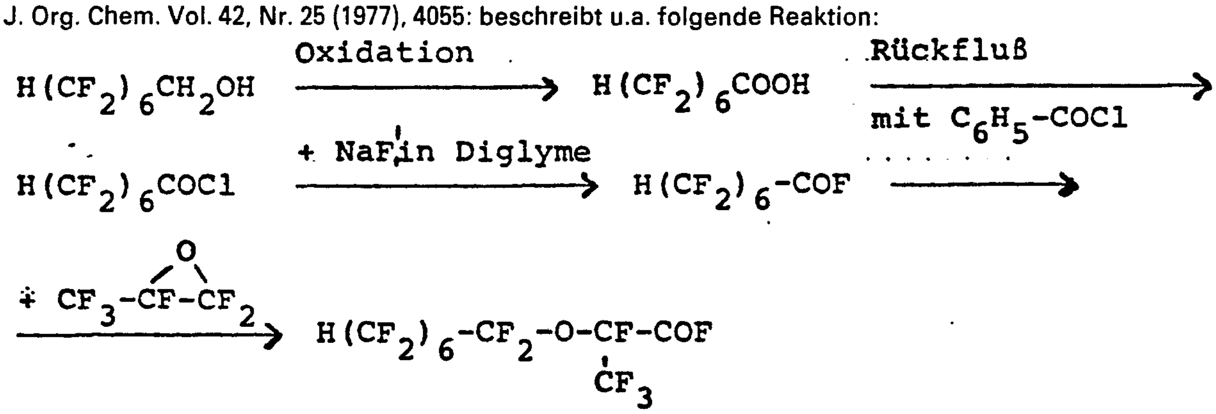


Die letzten drei Verbindungen dieser Reaktionsreihe sind sämtlich Verbindungen der Formel II; in ganz analoger Weise sind auch die anderen unter die Formel II fallenden Verbindungen erhältlich.

Die unter vorstehende Formel I fallenden ω-Fluorsulfatoperfluorcarbonsäureester - also die Verbindungen der Formel I':

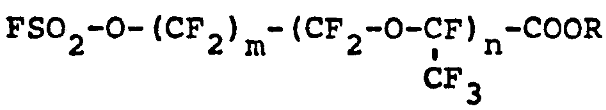 (I')

worin R = organischer Rest, insbesondere $CH_3$ oder $C_2H_5$, und m und n die gleiche Bedeutung wie in den Formeln I - III besitzen,

können dann etwa nach dem Verfahren der Patentanmeldung P 30 34 549.4 (HOE 80/F 197) in Gegenwart katalytischer Alkalifluorid-Mengen und in Abwesenheit von Lösungsmitteln in die entsprechenden perfluorierten Dicarbonsäurefluoridester der Formel V

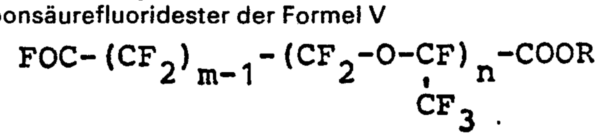 (V)

worin R, m und n die vorgenannte Bedeutung besitzen, umgewandelt werden.

Durch die an sich bekannte Reaktionsfolge:

Umsetzung mit Hexafluorpropenepoxid - Verseifung mit wässrigem Alkalihydroxid - Abspaltung von Alkalifluorid und Kohlendioxid durch Erhitzen sind aus den perfluorierten Dicarbonsäurefluoridestern der Formel V perfluorierte Vinylether mit noch einer Estergruppe am anderen Molekülende (= Verbindungen der Formel VI) erhältlich:

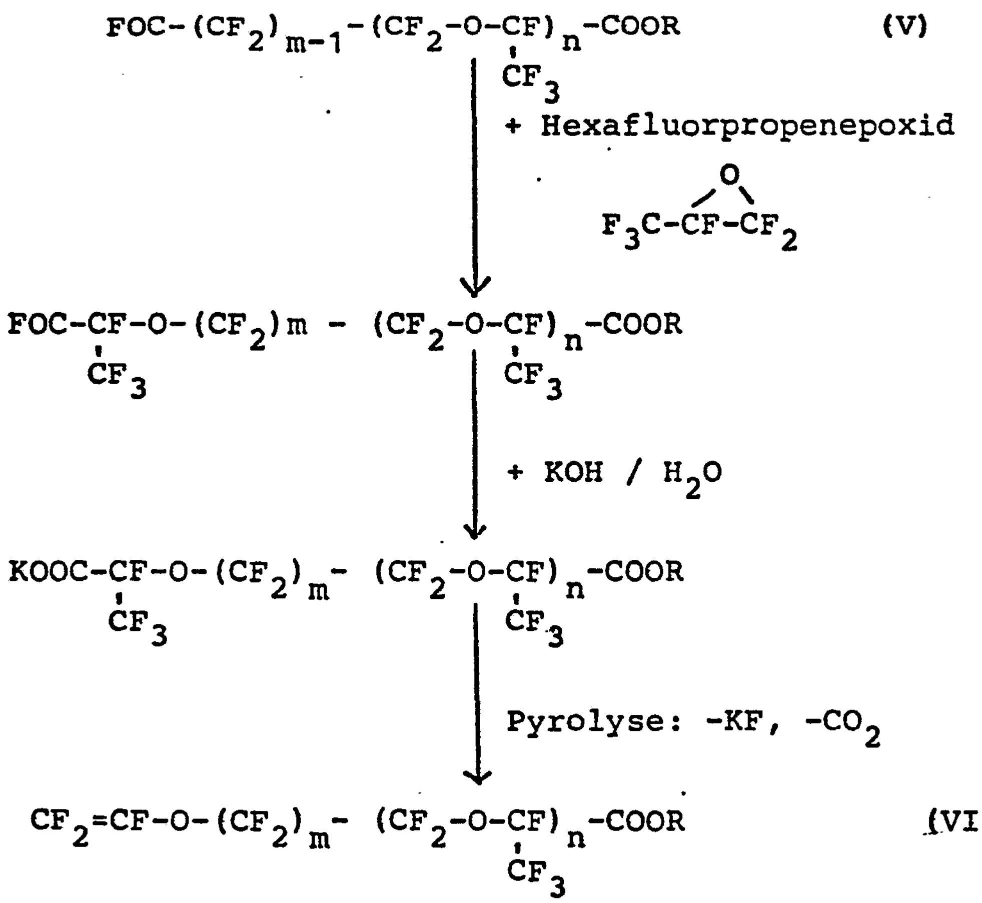


(V)

(VI)

Derartige perfluorierte Vinylether mit noch einer Estergruppe am anderen Molekülende sind wichtige Monomere, deren Copolymerisate mit Tetrafluorethylen und anderen Fluorolefinen zur Erzeugung von ionenselektiven Membranen, Kationenaustauschermassen und Fluorelastomeren verwendet werden.

Für die Herstellung der perfluorierten Vinylether mit noch einer funktionellen Gruppe am anderen Molekülende sind also nach dem Vorstehenden ω-Fluorsulfato-perfluorcarbonsäurederivate (insbesondere die Ester) bzw. perfluorierte Dicarbonsäurefluoridester wichtige Schlüsselsubstanzen.

Derartige perfluorierte aliphatische Verbindungen mit zwei verschiedenen funktionellen Gruppen im Molekül können im prinzip auch - ausgehend von perfluorierten aliphatischen α,ω-Bis-fluorsulfato-Verbindungen - durch Abspaltung der beiden Fluorsulfatogruppen etwa mit Cäsiumfluorid CsF und partielle Veresterung der bei der Abspaltung resultierenden Dicarbonsäuredifluoride erhalten werden; denn folgende Reaktionen sind bekannt:

1. J. Fluorine Chemistry 16,S. 63 - 73, insbesondere S. 65 (1980):

$$FSO_2\text{-}O\text{-}CF_2\text{-}CF_2\text{-}CF_2\text{-}O\text{-}SO_2F \xrightarrow{CsF} FOC\text{-}CF_2\text{-}COF + 2\ SO_2F_2$$

2. DE-OS 2 751 050:

Darstellung perfluorierter Dicarbonsäurefluoridester durch partielle Veresterung der entsprechenden perfluorierten Dicarbonsäuredifluoride mit Alkohol:

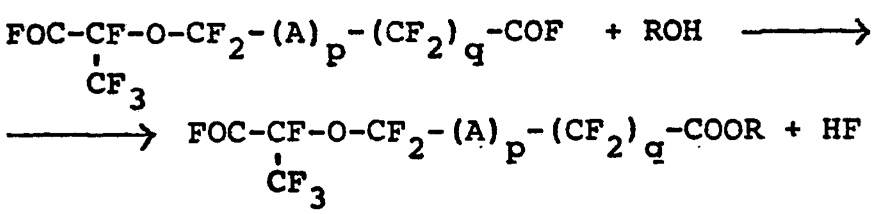

(A = bifunkt. $C_1$-$C_{10}$-perfluorgruppe, R = organ. Rest, p = 0 oder 1, q = 1 - 8)

Dieses partielle Veresterungsverfahren läuft jedoch nicht selektiv zu den perfluorierten Dicarbonsäurefluoridestern ab, sondern führt stets zu Gemischen mit den schwer abtrennbaren isomeren Halbestern sowie den Diestern, gegebenenfalls neben unumgesetztem Ausgangsprodukt.

Da die selektive Umwandlung nur einer funktionellen Gruppe in Molekülen mit 2 gleichen funktionellen

Gruppen meist nicht mit der gewünschten Vollständigkeit verläuft - es entstehen hier praktisch immer Gemische der gewünschten Verbindung mit einer umgewandelten funktionellen Gruppe mit der Verbindung mit beiden umgewandelten funktionellen Gruppen und der Ausgangsverbindung, was oft nur mit erheblichem Aufwand trennbar ist - erschien die Methode der partiellen Veresterung von perfluorierten Dicarbonsäuredifluoriden zur Herstellung von perfluorierten Dicarbonsäurefluoridestern nicht in dem gewünschten Maß aussichtsreich.

Die Schwierigkeiten bei der Umwandlung nur einer von zwei gleichen funktionellen Gruppen im Molekül gerade in der Fluorchemie werden beispielsweise auch deutlich aus der US-PS 4 181 679. Wie in dieser US-PS mehrfach betont wird, geht es deren Autoren dort um die Herstellung von ω-Jodperfluoralkylenoxidacylfluoriden durch selektive Reaktion nur einer von 2 gleichen $CF_2I$-Gruppen im entsprechenden Ausgangsmolekül zur COF-Gruppe durch Erhitzen mit Zinksulfat/Schwefelsäure auf Temperaturen von etwa 60 bis 120° C nach folgender schematischen Reaktionsgleichung:

$$JCF_2-CF_2-O-(CF_2)_X-O-CF_2-CF_2J \xrightarrow[60-120°C]{ZnSO_4/H_2SO_4}$$

$$JCF_2-CF_2-O-(CF_2)_X-O-CF_2-COF$$

(X = ganze Zahl von mindestens 2)

Wie insbesondere aus Beispiel 3 der PS hervorgeht, wird das gewünschte Produkt mit 2 ungleichen funktionellen Gruppen (nämlich der $CF_2J$ und der COF-Gruppe) im Molekül bestenfalls nur in einer Ausbeute von 35 % erhalten.

Obwohl der Weg zu den letztlich gewünschten perfluorierten Vinylethern mit noch einer weiteren funktionellen Gruppe (insbesondere der Estergruppe) im Molekül über die ω-Fluorsulfato-perfluorcarbonsäurederivate und die perfluorierten Dicarbonsäurefluoridester gemäß dem Verfahren der Patentanmeldungen P 30 34 538.1 und p 30 34 549.4 durchaus gangbar ist

und obwohl auch die vorstehend angedeutete Möglichkeit, ausgehend von α,ω-Bis-fluorsulfato-perfluoralkanen über die perfluorierten Dicarbonsäuredifluoride und deren partielle Veresterung einen gangbaren Weg darstellt, befriedigen diese Methoden teils wegen der nicht ganz einfachen Zugänglichkeit der Ausgangsprodukte (der Formel II für das Verfahren der erwähnten Patentanmeldungen), teils wegen der nicht ganz in dem gewünschten und erforderlichen Maß selektiven partiellen Veresterung der entsprechenden perfluorierten Dicarbonsäuredifluoride, nicht vollständig.

Es bestand daher die Aufgabe, einen verbesserten Weg zu den perfluorierten Vinylethern mit noch einer weiteren funktionellen Gruppe - insbesondere einer Estergruppe- im Molekül zu erschließen.

Diese Aufgabe konnte erfindungsgemäß durch eine verbesserte Darstellung der auf diesem Weg als Zwischenprodukte fungierenden ω-Fluorsulfato-perfluoralkansäurefluoride und -ester gelöst werden. Erfindungsgegenstand ist somit ein Verfahren zur Herstellung von ω-Fluorsulfato-perfluoralkansäurederivaten der Formel VII

$FSO_2$-0-$CF_2$-$R_f$-COY (VII)

worin $R_f$

ein unverzweigter oder verzweigter Perfluoralkylenrest mit 1 - 10, insbesondere 2 - 8 C-Atomen, und Y = F oder OR (R = Alkylrest vorzugsweise mit 1

- 10 C-Atomen, insbesondere $CH_3$ oder-$C_2H_5$)

ausgehend von ω-Fluorsulfatoverbindungen, dadurch gekennzeichnet, daß man α,ω-Bis-fluorsulfatoperfluoralkane der Formel VIII

$FSO_2$-O-$CF_2$-$R_f$-$CF_2$-O-$SO_2F$ (VIII),

worin $R_f$ die gleiche Bedeutung wie in Formel VII besitzt,

a) in Gegenwart katalytischer Mengen eines oder mehrerer Alkalifluoride und/oder Alkalihydrogenfluoride bei Temperaturen zwischen etwa -30 und +150° C unter ständiger Entfernung der zunächst entstehenden Verbindungen der Formel VII mit Y = F, oder

b) in Gegenwart katalytischer bis etwa äquimolarer Mengen eines oder mehrerer Alkalifluoride und/oder Alkalihydrogenfluoride

sowie in Gegenwart einer mindestens äquimolaren Menge eines Alkohols der Formel IX

ROH (IX)

worin R die bei Formel VII genannte Bedeutung besitzt,

und gegebenenfalls auch eines inerten, die Alkalifluoride und/oder -hydrogenfluoride nicht lösenden Verdünnungsmittels,

im Temperaturbereich ebenfalls zwischen etwa -30 und +150° C zu Verbindungen der Formel VII mit Y = OR bis zur Freisetzung der etwa äquimolaren Menge $SO_2F_2$ umsetzt.

Durch diese an sich einfache Verfahrensweise gelingt es, aus den gut zugänglichen α,ω-Bis-fluorsulfato-perfluoralkanen der Formel VIII selektiv nur eine der beiden Fluorsulfatogruppen mit Ausbeuten bis zu etwa 70 % d.Th. (unter Bildung der Säurefluorid-oder der Estergruppe) abzuspalten. Dieses Ergebnis war im Hinblick auf die nur unzureichende Selektivität bei den bekannten Verfahren der Umsetzung von nur einer von zwei

gleichen, in ein und demselben Molekül vorhandenen funktionellen Gruppen insbesondere auf dem Gebiet der fluororganischen Verbindungen (vgl. DE-OS 2 751 050 und US-PS 4 181 679) nicht zu erwarten und daher außerordentlich überraschend.

Die Ausgangssubstanzen für das erfindungsgemäße Verfahren - die α,ω-Bis-fluorsulfato-perfluoralkane der Formel VIII - können beispielsweise nach folgenden Methoden erhalten werden:

1. J. M. Shreeve und G.H. Cady, J. Am. Chem. Soc. Vol 83, S. 4521 bis 4525, insbesondere S. 4523 (1961): Gasphasenreaktion von Tetrafluorethylen mit Peroxodisufuryl-difluorid $FSO_2$-O-O-$SO_2F$ zu 1,2-Bis-fluorsulfato-tetrafluorethan:

$$CF_2{=}CF_2 + FSO_2\text{-}O\text{-}O\text{-}FSO_2F \rightarrow FSO_2\text{-}O\text{-}CF_2\text{-}CF_2\text{-}O\text{-}SO_2F$$

2. C.G.Krespan, J. Fluorine Chemistry 2, S. 173 bis 179, insbesondere S. 174 (1972/73): Gasphasenreaktion von Hexafluorpropen mit Peroxodisulfuryldifluorid zu einem Gemisch der entsprechenden 1:1 (62 %) und 2:1 (22 %) Addukte:

$$CF_2{=}CF\text{-}CF_3 + FSO_2\text{-}O\text{-}O\text{-}SO_2F \longrightarrow FSO_2\text{-}O\text{-}CF_2\text{-}\underset{\displaystyle CF_3}{\underset{|}{C}F}\text{-}O\text{-}SO_2F \quad (62\ \%)$$

$$+ \; FSO_2\text{-}O\text{-}(C_3F_6)_2\text{-}O\text{-}SO_2F \quad (22\%)$$

3. C.J. Shack und K. O. Christe, J. Fluorine Chemistry 16, S. 73 (1980): Reaktion von α,ω-Bis-bromalkanen mit Chlor-fluorsulfat zu den entsprechenden α,ω-Bis-fluorsulfato-perfluoralkanen, z.B.

$$Br\text{-}CF_2\text{-}CF_2\text{-}CF_2\text{-}Br + 2\,ClOSO_2F \rightarrow FSO_2\text{-}O\text{-}(CF_2)_3\text{-}O\text{-}SO_2F + 2\,BrCl$$

4. A. Germain und A. Commeyras, Tetrahedron Vol. 37, S. 487 bis 491: Anodische Oxidation von α,ω-Bis-jod-perfluoralkanen in einem Elektrolyten aus einer Lösung von Alkalifluorid in Fluorsulfonsäure. Die Autoren sind der Meinung, daß ein direkter Elektrodenprozeß vorliegt, schließen aber einen gleichzeitigen, indirekten Prozeß über "I+" nicht aus (S. 488, rechte Spalte).

5. Nach dem Verfahren der gleichzeitig eingereichten Patentanmeldung P (HOE 81/F 174) werden α,ω-Bisfluorsulfato-perfluoralkane besonders vorteilhaft aus perfluorierten α-Olefinen und Peroxodisulfuryl-difluorid dadurch erhalten,

daß man die perfluorierten α-Olefine in eine das Peroxodisulfuryl-difluorid enthaltende flüssige Phase einleitet, wobei man die Konzentration des Peroxodisulfuryl-difluorids in der flüssigen Phase innerhalb des Konzentrationsbereiches von etwa 0,005 bis 0,2, vorzugsw. von etwa 0,01 bis 0,1 Mol/l im wesentlichen konstant hält. In einer bevorzugten Ausführungsform dieses Verfahrens werden die perfluorierten α-Olefine in die flüssige Phase einer Elektrolysezelle eingeleitet, in welcher durch Elektrolyse einer Lösung von Alkalifluorsulfonat in Fluorsulfonsäure Peroxodisulfuryl-difluorid gebildet und im Ausmaß von dessen Verbrauch laufend nachgeliefert wird.

Das Verfahren liefert hauptsächlich die 2:1-Addukte aus den entsprechenden perfluorierten α-Olefinen und Peroxodisulfuryl-difluorid. Mit Tetrafluorethylen als Ausgangs-Olefin entsteht daher so als Hauptprodukt 1,4-Bis-fluorsulfato-perfluorbutan:

$$2\,CF_2{=}CF_2 + FSO_2\text{-}O\text{-}O\text{-}SO_2F \rightarrow FSO_2\text{-}O\text{-}(CF_2)_4\text{-}O\text{-}SO_2F$$

Katalysatoren sind beim erfindungsgemäßen Verfahren die Alkalifluoride und/oder Alkalihydrogenfluoride, wobei die Na- und K-Verbindungen bevorzugt sind.

Im Falle der Variante a) des erfindungsgemäßen Verfahrens ist eine katalytische Menge des Alkalifluorids und/oder Alkalihydrogenfluorids - im allgemeinen zwischen etwa 0,1 und 30 Mol-%, bezogen auf das Ausgangs-Bisfluorsulfato-perfluoralkan VIII, ausreichend.

Die Verwendung eines Lösungsmittels ist hier in der Regel nicht zweckmäßig.

Als Reaktionstemperaturen kommen im prinzip Temperaturen zwischen etwa -30 und +150°C in Frage, wobei Temperaturen zwischen etwa 20 und 120°C bevorzugt sind.

Es kann sowohl bei Normaldruck als auch bei Unter- oder Überdruck gearbeitet werden.

Bei der Reaktionsdurchführung ist es praktisch ohne Belang, in welcher Reihenfolge die Reaktionskomponenten zusammengegeben werden. Es ist allerdings von Vorteil, während der gesamten Dauer der Reaktion durch Rühren für eine gute Durchmischung des Ansatzes zu sorgen. Es ist bevorzugt, das Ausgangs-Bisfluorsulfato-perfluoralkan und den Katalysator zusammenzugeben und gegebenenfalls zu erhitzen, bis Gasentwicklung eintritt. Es ist hier besonders wichtig und wesentlich, daß das entstehende ω-Fluorsulfato-perfluoralkansäurefluorid - d.i. die Verbindung der Formel VII mit Y = F - während der Reaktion ständig entfernt wird; dies geschieht zweckmäßig durch kontinuierliche Abdestillation über eine Kolonne. Dabei ist der Druck zweckmäßig so einzustellen, daß der Siedepunkt des abdestillierenden Produktes etwa 10 bis 60°C unter der Reaktionstemperatur liegt.

In Verfahrensvariante b) kann eine katalytische bis etwa äquimolare Menge Alkalifluorid und/oder Alkalihydrogenfluorid - im allgemeinen zwischen etwa 0,1 und 100 Mol-%, vorzugsweise zwischen etwa 1 und 30 Mol-%, bezogen auf das Ausgangs-Bisfluorsulfato-perfluoralkan VIII - verwendet werden.

Die Temperatur- und Druckbedingungen dieser Verfahrensvariante entsprechen praktisch denen der Variante a).

Im Falle b) ist es jedoch zweckmäßig, außer einer mindestens etwa äquimolaren Menge des als Reaktionspartner notwendigen Alkohols der Formel IX auch noch ein inertes, die Alkalifluoride und/oder -hydrogenfluoride nicht lösendes Verdünnungsmittel zuzusetzen. Als solche Verdünnungsmittel können z.B. halogenierte Kohlenwasserstoffe wie Methylenchlorid etc. verwendet werden.

Eine zweckmäßige Durchführungsweise der Variante b) besteht z.B. darin, das Ausgangs-Bisfluorsulfato-perfluoralkan VIII, den Alkohol IX und das Alkalifluorid und/oder -hydrogenfluorid sowie gegebenenfalls das Verdünnungsmittel bei tiefer Temperatur zusammenzugeben und langsam zu erwärmen bzw. erwärmen zu lassen. Nachdem etwa die berechnete Menge an Sulfurylfluorid $SO_2F_2$ entstanden ist (Messung etwa mittels einer Gasuhr!), filtriert man das Alkalifluorid und/oder -hydrogenfluorid ab, wäscht mit Wasser Alkohol- und säurefrei, trocknet und destilliert.

Nach dieser Variante werden die ω-Fluorsulfato-perfluorcarbonsäureester der Formel VII mit Y = OR erhalten.

Falls beabsichtigt ist, diesen Ester etwa durch Abspaltung der noch verbliebenen Fluorsulfatogruppe mittels Alkalifluorid zum entsprechenden Perfluoralkan-dicarbonsäurefluoridester weiter zu verarbeiten, ist es von Vorteil, bei der Aufarbeitung des Ansatzes der Verfahrensvariante b) am Ende keine fraktionierte Destillation vorzunehmen, sondern dann erst den Perfluoralkan-dicarbonsäurefluoridester zu fraktionieren.

Da insbesondere bei Verfahrensvariante b) als Reaktionsprodukt auch Flußsäure entsteht, ist es besonders hier ratsam, in Gefäßen aus flußsäurebeständigem Material zu arbeiten.

Wegen der relativ einfach und gut zugänglichen Ausgangs-Bis-fluorsulfato-perfluoralkane der Formel VIII, der außerordentlich einfachen Durchführbarkeit und der hohen Selektivität und Ausbeute (bis etwa 70 % d.Th.) des erfindungsgemäßen Verfahrens stellt dieses einen erheblichen Fortschritt auf diesem Gebiet dar.

Die Erfindung wird nun durch die nachfolgenden Beispiele näher erläutert.

Nach den Erfindungsbeispielen A folgen noch Weiterverarbeitungsbeispiele B, welche sich auf die Weiterverarbeitung der nach dem erfindungsgemäßen Verfahren hergestellten ω-Fluorsulfatoperfluoralkansäurederivate beziehen.

Alle Reaktionen werden in einem Abzug durchgeführt:

$^1H$-NMR-Spektren: Lösungsmittel $CDCl_3$, TMS $[(CH_3)_4Si]$
als innerer Standard

$^{19}F$-NMR-Spektren: Lösungsmittel $CDCl_3$; $CFCl_3$ als innerer Standard

## A)-Erfindungsbeispiele

Aa) Verfahrensvariante a:
Herstellung von ω-Fluorsulfato-perfluoralkansäurefluoriden

### Beispiel 1

4-Fluorsulfato-perfluorbutansäurefluorid $FSO_2$-O-$(CF_2)_3$-COF

In einem trockenen Kolben mit Magnetrührer, Thermometer, Vigreuxkolonne, Kolonnenkopf und nachgeschalteter Kältefalle (-78°C) legt man 2,9 g (0.05 Mol) trockenes Kaliumfluorid und 120 g (0.3 Mol) 1,4-Bisfluorsulfato-perfluorbutan vor. Man erhitzt auf 80 - 100°C. Unter kräftiger Gasentwicklung geht farblose Flüssigkeit über, die, ohne Rückfluß aufkommen zu lassen, abgenommen wird. Nach der Reaktion befinden sich 56 g Flüssigkeit in der Kältefalle. Davon sind bei +10°C 38 g flüchtig. Der Rückstand wird zusammen mit dem Destillat fraktioniert. Dabei werden 10 g (17 %) Perfluorbutandisäuredifluorid und 45,5 g (51 %) 4-Fluorsulfato-perfluorbutansäurefluorid erhalten (Kp 83-85°C/755 mm)

Analyse: Ber. C 16,23 F 51,31 S 10,83

Gef. C 16,3 F 51,3 S 11,5

$^{19}F$-NMR: +51.1 (t, 1F, -O-$SO_2F$), +25.3 (m, 1F, -COF), -83.3 (q, 2F, -$CF_2$-O-), -118.6 (q, 2F, -$CF_2$-CO-), -125.7 (s, 2F, -$CF_2$-)

IR (Gasspektrum): 5.28 μ (C=O), 6.63 μ (S=O)

### Beispiel 2

4-Fluorsulfato-perfluorbutansäurefluorid $FSO_2$-O-$(CF_2)_3$-COF

In eine wie in Beispiel 1 beschriebene Apparatur gibt man 398 g (1.0 Mol) 1,4-Bisfluorsulfato-perfluorbutan, 1,16 g (0.02 Mol) trockenes Kaliumfluorid und 0,84 g (0.02 Mol) Natriumfluorid. Anschließend wird auf 100 - 120°C erhitzt. Nach kurzer Zeit setzt Gasentwicklung ein und es tritt Rückfluß in der Kolonne auf. Die übergehende Flüssigkeit weist einen Siedepunkt von 75 - 80°C auf. Die Aufarbeitung erfolgt wie bei Beispiel 1. Man erhält 196 g (66 %) 4-Fluor-sulfato-perfluorbutansäurefluorid.

**Beispiel 3**

6-Fluorsulfato-perfluorhexansäurefluorid $FSO_2$-O-$(CF_2)_5$-COF

In eine unter Beispiel 1 beschriebene Apparatur gibt man 200 g (0.4 Mol) 1,6-Bisfluorsulfato-perfluorhexan, 1,16 g (0.02 Mol) Kaliumfluorid und 1,68 g (0.04 Mol) Natriumfluorid. Bei 200 Torr erhitzt man das Gemisch, bis Gasentwicklung eintritt. Das dabei übergehende Destillat wird so abgenommen, daß der Siedepunkt 90°C/200 mm nicht übersteigt. Die Aufarbeitung erfolgt wie bei Beispiel 1. Mit Kp 73 - 76°C/ 740 Torr werden 11 g (9 %) Perfluorhexandisäuredifluorid und mit Kp 126 - 127°C/740 mm werden 92,5 g (58 %) 6-Fluor-sulfatoperfluorhexansäurefluorid erhalten.

Analyse: Ber. C 18,19 F 57,56 S 8,09

Gef. C 18,30 F 57,10 S 9,50

$^{19}$F-NMR: + 50.6 (t, IF, -O-$SO_2$F), +24,8 (m, IF, -COF), -83.3 (m, 2F, -O-$CF_2$-), -118.4 (m, 2F, -$CF_2$-CO-), -121.9 (m, 2F, $CF_2$), -122.8 (m, 2F, $CF_2$), -125.0 (m, 2F, $CF_2$)

IR (neat): 5.31 μ (C=O), 6. 68 μ (S=O)

**Beispiel 4**

8-Fluorsulfato-perfluoroctansäurefluorid $FSO_2$-O-$(CF_2)_7$-COF

Nach der Arbeitsvorschrift des Beispiels 3 werden 100 g (0.17 Mol) 1,8-Bisfluorsulfato-perfluoroctan mit 0,58 g (0.01 Mol) Kaliumfluorid und 0,84 g (0.02 Mol) Natriumfluorid bei 50 mm erhitzt. Die Destillation über eine Füllkörperkolonne liefert 42 g (51 %) 8-Fluorsulfato-perfluoroctansäurefluorid mit Kp. 86 - 87°C (50 mm).

Analyse: Ber. C 19,37 F 61,27 S 6,46

Gef. C 19,60 F 60,20 S 7,50

$^{19}$F-NMR: +50.6 (t, 1F, -O-$SO_2$F), +24.7 (m, 1F, -COF), -83.4 (m, 2F, -$CF_2$-O-), -118.7 (m, 2F, -$CF_2$-CO-), -122.2 (m, 6F, $CF_2$), -122,8 (m, 2F, $CF_2$), -125.0 (m, 2F, $CF_2$)

IR (neat): 5.31 μ (C=O), 6.67 μ (S=O)

Ab) Verfahrensvariante b:

Herstellung von ω-Fluorsulfato-perfluoralkansäureestern, die hier allerdings durch Erhitzen mit Akalifluoriden (→ Abspaltung der Fluorsulfatogruppe) gleich weiter zu den entsprechenden Perfluoralkandisäurefluoridestern umgesetzt werden.

**Beispiel 5**

4-Fluorsulfato-perfluorbutansäuremethylester

$FSO_2$-O-$CF_2$-$CF_2$-$CF_2$-$COOCH_3$ mit weiterer Umsetzung zu 3-Carbomethoxy-perfluorpropansäurefluorid FOC-$CF_2$-$CF_2$-$COOCH_3$

In einen trockenen Kolben mit Magnetrührer, Thermometer, Rückflußkühler und Blasenzähler gibt man bei -30°C 250 ml Methanol, 25,2 g (0.6 Mol) Natriumfluorid und 240 g (0.6 Mol) 1,4-Bisfluorsulfato-perfluorbutan. Unter gutem Rühren läßt man das Gemisch langsam auf Raumtemperatur kommen. Unter Abspaltung von Sulfurylfluorid setzt sich das Bisfluorsulfat um. Man rührt bei RT nach, bis die untere Phase verschwunden ist. Nach dem Abfiltrieren des ausgefallenen Salzes gießt man auf Eiswasser. Die organische Phase wird mit Wasser säurefrei gewaschen und über Calciumchlorid getrocknet. Die Destillation über eine kurze Vigreux-Kolonne liefert 159 g farblose Flüssigkeit mit Kp 142 - 177°C/760 mm, die nach der Vorschrift des Beispiels 4 mit 1.8 g (0.03 Mol) Kaliumfluorid umgesetzt werden. Die Destillation ergibt 81 g (65 % bezogen auf das eingesetzte Bisfluorsulfat) 3-Carbomethoxyperfluorpropansäurefluorid neben geringen Mengen an Perfluorbutandisäuredifluorid und Perfluorbutandisäuredimethylester.

**Beispiel 6**

4-Fluorsulfato-perfluorbutansäuremethylester $FSO_2$-O-$CF_2$-$CF_2$-$CF_2$-$COOCH_3$ mit weiterer Umsetzung zu 3-Carbomethoxyperfluorpropansäurefluorid FOC-$CF_2$-$CF_2$-$COOCH_3$

Nach der Arbeitsvorschrift des Beispiels 5 setzt man 100 g (0.25 Mol) 1,4-Bisfluorsulfato-perfluorbutan mit 3,9 g (0.05 Mol) Kaliumhydrogenfluorid in einem Gemisch von 100 ml Methanol und 100 ml Methylenchlorid bei anfangs 0-10°C,später bei ca. 20°C um. Nach dem Ende der Gasentwicklung filtriert man das Salz ab und arbeitet wie in Beispiel A5 beschrieben auf. Man erhält 62 g Destillat mit Kp 139 - 176°C/755 mm, das nach der Umsetzung mit 0,58 g (0.01 Mol) Kaliumfluorid 24 g (46 %) 3-Carbomethoxyperfluorpropansäurefluorid ergibt.

**Weiterverarbeitungsbeispiele**

**Beispiel 1**

4-Fluorsulfato-perfluorbutansäuremethylester $FSO_2\text{-}O\text{-}(CF_2)_3\text{-}COOCH_3$

Zu einer Lösung von 280 g (0.95 Mol) 4-Fluorsulfato-perfluorbutansäurefluorid in 250 ml Methylenchlorid, die bei 0 - 10°C gehalten wird, tropft man unter gutem Rühren eine Lösung von 35,2 g (1.1 Mol) Methanol in 50 ml Methylenchlorid. Der Ansatz rührt eine Stunde nach. Anschließend wird mit Wasser säurefrei gewaschen und die organische Phase über Calciumchlorid getrocknet. Bei der Destillation über eine Füllkörper-Kolonne werden mit Kp 71 - 73°C/50 mm 260 g (89 %) 4-Fluorsulfatoperfluorbutan-säuremethylester erhalten.

Analyse: Ber. C 19,49 H 0,98 F 43,16 S 10,41
Gef. C 19,8 H 1,0 F 42,7 S 10,6
$^1$H-NMR: 3.98 (s, $-O-CH_3$)
$^{19}$F-NMR: + 50.5 (t, 1F, $-O-SO_2F$), -83.5 (q, 2F, $-O-CF_2$), -119.0 (t, 2F, $-CF_2-CO-$), -126.2 (s, 2F, $CF_2$)
IR (neat): 3.41 μ (CH), 5.55 μ (C=O), 6.68μ(S=O)

**Beispiel 2**

6-Fluorsulfato-perfluorhexansäuremethylester $FSO_2\text{-}O\text{-}(CF_2)_5\text{-}COOCH_3$

Wie in Beispiel B1 beschrieben setzt man eine Lösung von 92 g (0.23 Mol) 5-Fluorsulfatoperfluorhexansäurefluorid in 75 ml Methylenchlorid mit einer Lösung von 9,6 g (0.3 Mol) Methanol in 75 ml Methylenchlorid um. Man erhält 81 g (85 %) 6-Fluorsulfatoperfluorhexansäuremethylester mit einem Siedepunkt von 75 - 77°C/20 mm.

Analyse: Ber. C 20,60 H 0,74 F 51,20 S 7,86
Gef. C 20,3 H 0,5 F 50,9 S 8,3
$^1$H-NMR: 3,97 (s, $-O-CH_3$)
$^{19}$F-NMR: +50.5 (t, IF, $-O-SO_2F$, J = 8Hz), -83.4 (m, 2F, $-CF_2-O-$), -118.8 (m, 2F, $-CF_2-CO-$), -122.2 (m, 2F, $CF_2$), -123.5 (m, 2F, $CF_2$), -124.9 (m, 2F, $CF_2$)
IR (neat): 3.42 μ (CH), 5.68 μ (C=O), 6.69 μ (S=O)

**Beispiel 3**

8-Fluorsulfato-perfluoroctansäuremethylester $FSO_2\text{-}O\text{-}(CF_2)_7\text{-}COOCH_3$

Nach der Arbeitsvorschrift in Beispiel B1 setzt man eine Lösung von 5,5 g (0.17 Mol) Methanol in 100 ml Methylenchlorid bei 0 bis 10°C mit 78 g (0.16 Mol) 8-Fluorsulfato-perfluoroctansäurefluorid um. Die Destillation ergibt 72 g (90 %) 8-Fluorsulfatoperfluoroctansäuremethylester mit Kp 86 - 87°C/10 mm.

Analyse: Ber. C 21,27 H 0,60 F 56,08 S 6,31
Gef. C 21,2 H 0,6 F 55,9 S 5,9
$^1$H-NMR: 3.96 (s, $-O-CH_3$)
$^{19}$F-NMR: +50.7 (t, 1F, $-OSO_2F$, J=8 Hz), -83.3 (m, 2F, $-O-CF_2-$), -118.8 (m, 2F, $-CF_2-CO-$), -122.2 (m, 6F, $-CF_2-$), -123.0 (m, 2F, $-CF_2-$), -126.0 (m, 2F, $-CF_2-$)
IR (neat): 3.41 μ (CH), 5.56 μ (C=O), 6.68 μ (S=O)

**Beispiel 4**

3-Carbomethoxy-perfluorpropansäurefluorid $CH_3OOC\text{-}(CF_2)_2\text{-}COF$

In einem trockenen Kolben mit Magnetrührer, Thermometer, Rückflußkühler und Blasenzähler legt man 260 g (0.84 Mol) 4-Fluorsulfato-perfluorbutansäurefluorid und 2,9 g (0.05 Mol) trockenes Kaliumfluorid vor. Man erhitzt das Gemisch, bis Gasentwicklung eintritt. Die Abspaltung des Sulfurylfluorids erfolgt zwischen 70 und 100°C. Nach dem Ende der Gasentwicklung wird die im Kolben verbliebene Flüssigkeit über eine Füllkörperkolonne destilliert. Mit Kp 98 - 99°C /755 mm erhält man 169 g (97 %) 3-Carbomethoxyperfluorpropansäurefluorid.

**Beispiel 5**

5-Carbomethoxyperfluorpentansäurefluorid $CH_3OOC-(CF_2)_4-COF$

Nach der Vorschrift des Beispiel B4 setzt man 130 g (0.32 Mol) 6-Fluorsulfato-perfluorhexansäuremethylester mit 0,58 g (0.01 Mol) trockenem Kaliumfluorid um. Die Abspaltung des Sulfurylfluorids erfolgt zwischen 80 und 120° C. Die Destillation liefert 94 g (96 %) 5-Carbomethoxyperfluor-pentansäurefluorid mit Kp 62 - 63° C/48 mm.

**Beispiel 6**

7-Carbomethoxy-perfluorheptansäurefluorid $CH_3OOC-(CF_2)_6-COF$

Nach der Arbeitsvorschrift des Beispiels B4 erhitzt man 67 g (0.13 Mol) 8-Fluorsulfatoperfluoroctansäuremethylester mit 0,58 g (0.01 Mol) Kaliumfluorid. Die Abspaltung des Sulfurylfluorids setzt bei ca. 80° C ein. Die nachfolgende Destillation ergibt 47 g (88 %) 7-Carbomethoxyperfluor-heptansäurefluorid mit Kp 88 - 90° C/50 mm.

$^1$H-NMR: 3.94 (s)

$^{19}$F-NMR: +24.9 (m, 1F, COF), -118.8 (m, 4F, $-CF_2-CO-$), -122.1 (m, 4F, $CF_2$), -123.1 (m, 4F, $CF_2$)

IR (neat): 5.31 μ (COF), 5.57 μ (-COO-)

**Patentansprüche**

1. Verfahren zur Herstellung von ω Fluorsulfato-perfluoralkansäurederivaten der Formel VII

$FSO_2-O-CF_2-R_f-COY$ (VII)

worin $R_f$

ein unverzweigter oder verzweigter Perfluoralkylenrest vorzugsweise mit 1 - 10, insbesondere mit 2 - 8 C-Atomen

Y = F oder OR (R = Alkylrest vorzugsweise mit 1 - 10 C-Atomen, insbesondere $CH_3$ oder $C_2H_5$)

ausgehend von ω-Fluorsulfato-Verbindungen, dadurch gekennzeichnet, daß man α,ω-Bisfluorsulfatoperfluoralkane der Formel VIII

$FSO_2-O-CF_2-R_f-CF_2-O-SO_2F$ (VIII)

worin $R_f$ die gleiche Bedeutung wie in Formel VII besitzt,

a) in Gegenwart katalytischer Mengen eines oder mehrerer Alkalifluoride und/oder Alkalihydrogenfluoride bei Temperaturen zwischen etwa -30 und +150° C unter ständiger Entfernung der entstehenden Verbindungen der Formel VII mit Y = F, oder

b) in Gegenwart katalytischer bis etwa äquimolarer Mengen eines oder mehrerer Alkalifluoride und/oder Alkalihydrogenfluoride

sowie in Gegenwart einer mindestens etwa äquimolaren Menge eines Alkohols der Formel IX

ROH (IX)

worin R die bei Formel VII genannte Bedeutung besitzt,

und gegebenenfalls auch eines inerten, die Alkalifluoride und/oder -hydrogenfluoride nicht lösenden Verdünnungsmittels zu Verbindungen der Formel VII mit Y = OR bis zur Freisetzung einer der Menge der Ausgangsverbindung VIII etwa äquimolaren Menge Sulfurylfluorid $SO_2F_2$ umsetzt.

2. Verfahren nach Anspruch 1, dadurch gekennzeichnet, daß man in Teil a) des Verfahrens die Entfernung der Verbindung der Formel VII mit Y = F durch Abdestillation bei etwa 10 bis 60° C unter der Reaktionstemperatur vornimmt.

3. Verfahren nach einem der Ansprüche 1 bis 2, dadurch gekennzeichnet, daß man Teil a) des Verfahrens in Abwesenheit von Lösungsmitteln durchführt.

**Claims**

1. A process for the preparation of ω-fluorosulfatoperfluoroalkanoic acid derivatives of the formula VII

$FSO_2-O-CF_2-R_f-COY$ (VII)

in which $R_f$ is an unbranched or branched perfluoroalkylene radical, preferably having 1 -10, in particular 2 - 8, C atoms, and Y is F or OR (R is an alkyl radical preferably containing 1 - 10 C atoms, in particular $CH_3$ or $C_2H_5$), starting from ω-fluorosulfato compounds, characterized in reacting α,ω-bis fluorosulfatoperfluoroalkanes of the formula VIII

$FSO_2-O-CF_2-R_f-CF_2-O-SO_2F$ (VIII)

in which $R_f$ has the same meaning as in formula VII,

a) in the presence of catalytic amounts of one or more alkali metal fluorides and/or alkali metal hydrogen

fluorides at temperatures between about -30 and +150° C, with continuous removal of the resulting compounds of the formula VII in which Y is F, or

b) in the presence of catalytic to approximately equimolar amounts of one or more alkali metal fluorides and/or alkali metal hydrogen fluorides and in the presence of an at least approximately equimolar amount of an alcohol of the formula IX

ROH (IX)

in which R has the meaning mentioned for formula VII, and, if appropriate, also in the presence of an inert diluent which does not dissolve the alkali metal fluorides and/or alkali metal hydrogen fluorides, to give compounds of the formula VII in which Y is OR, until an amount of sulfuryl fluoride $SO_2F_2$ which is approximately equimolar in relation to the amount of the starting compound VIII has been liberated.

2. The process as claimed in claim 1, characterized in effecting in part a) of the process, the removal of the compound of the formula VII in which Y is F by distilling off the latter at a temperature about 10 to 60° C less than the reaction temperature.

3. The process as claimed in either of claims 1 or 2, characterized in carrying out part a) of the process in the absence of solvents.

**Revendications**

1. Procédé de préparation de dérivés d'ω fluorosulfato-perfluoro-alcanoïques de formule VII

$FSO_2$- O-$CF_2$-$R_f$-COY (VII)

dans laquelle

$R_f$ représente un radical perfluoroalkylène ramifié ou non, ayant de 1 à 10 atomes de carbone, en particulier de 2 à 8, et

Y représente F ou OR (R étant un alkyle de préférence en $C_1$ à $C_{10}$, en particulier le groupe $CH_3$ ou $C_2H_5$)) à partir de composés d'ω-fluorosulfato, procédé caractérisé en ce que l'on transforme des α,ω-bisfluorosulfato-perfluoroalcanes de formule VIII

$FSO_2$-O-$CF_2$-$R_f$-$CF_2$-O-$SO_2F$ (VIII)

a) en présence de quantités catalytiques d'un ou de plusieurs fluorures et/ou hydrogénofluorures alcalins à des températures d'environ -30 à +150° C, avec élimination continue des composés d'abord formés de formule VII dans lesquels Y = F, ou bien

b) en présence de quantités catalytiques à environ équimolaires d'un ou de plusieurs fluorures et/ou hydrogénofluorures alcalins,

ainsi qu'en présence d'une proportion au moins équimolaire d'un alcool de formule IX:

ROH (IX)

dans laquelle R a la même signification que dans la formule VII,

et le cas échéant aussi d'un diluant inerte qui ne dissout pas les fluorures et hydrogénofluorures alcalins, en composés de formule VII dans lesquels Y = OR, jusqu'à libération d'une quantité de fluorure de sulfuryle $SO_2F_2$ sensiblement équimolaire par rapport à la quantité du composé de départ VIII.

2. Procédé selon la revendication 1 caractérisé en ce que dans la partie a) du procédé, l'élimination du composé de formule VII dans lequel Y est le fluor se fait par distillation à une température inférieure d'environ 10 à 60° C à la température de réaction.

3. Procédé selon les revendications 1 et 2 caractérisé en ce que l'on effectue la partie a) sans solvant.